# EUROPEAN PATENT APPLICATION

(11) **EP 1 604 744 A2**
(43) Date of publication of application: **14.12.2005**
(21) Application number: 05104991.4
(22) Date of filing: 08.06.2005
(51) Int. Cl.: B05B 17/04

(54) **Dispenser**

(30) Priority: 08.06.2004 EP 04253401
(71) Applicant: Tyco Electronics UK Limited, Swindon, Wiltshire SN3 5HH (GB)
(72) Inventor: Bowyer, John, SN25 1SH, Swindon (GB)
(74) Representative: Johnstone, Douglas Ian

(57) **Abstract**

A dispenser (2) dispenses small quantities of fluid from a tubular reservoir (60). An outer shell (38) and a core (72), which is rotatably mounted in the outer shell (38), form part of a fixed plate assembly (4). Fluid (120) is forced from the tubular reservoir (60) by a plunger (62) through a fluid inlet (52) of the outer shell (38) into a bore (84) in the core (72) thus displacing a piston (92) along the bore (84). The core (72) is then rotated so that a nozzle (90) at an end of the bore (84) is aligned with a fluid outlet (54) in the outer shell (38), and the piston (92) is displaced so as to eject the fluid from the bore (84) onto an active portion of an apertured member (18) forming part of a moving plate assembly (6) which is then displaced so as to move the active portion (18) away from the nozzle and thereby cleanly separate the dispensed fluid from the nozzle (90).

## Description

The present invention relates to a dispenser suitable for dispensing very small measured quantities of liquid, paste or grease.

When a measured quantity of a droplet or sub-droplet of liquid, paste or grease (hereinafter referred to as fluid) is to be dispensed, a number of problems occur. It is often difficult to separate the dispensed fluid from a nozzle through which it has been delivered without leaving more than a trace of the fluid on the nozzle. So-called stringing may occur leaving a trail of fluid connected to the nozzle. Air and also possibly part of the fluid delivered may be drawn into a device used to measure out the fluid. Lastly, it is difficult to repeatedly dispense the same volume of fluid consistently. These problems result from the surface tension and viscosity of the fluid and are exacerbated when very small volumes, such as 1 microliter, are being dispensed. One object of the invention is to provide a dispenser and method which overcome at least some of these problems.

One application in which it is necessary to dispense a very small volume of fluid is when a small volume of fluid, for example, medicament, perfume, insecticide or fungicide, is to be measured out and subsequently heated so as to vaporize the fluid. Vegetable or plant extracts for such purposes may be administered in such a manner but the problems mentioned above make the delivery of an appropriate volume of the extract onto a suitable heating member difficult. Accordingly, a further aspect of the invention is to provide a dispenser which is suitable for dosing out and vaporizing a small volume of fluid.

Thus, according to a first aspect of the invention, there is provided a measured droplet or sub-droplet dispenser comprising a fluid delivery member for measuring out a quantity of fluid having a nozzle through which the quantity of the fluid is delivered. The dispenser further comprises a fluid removal member for taking up the delivered quantity of the fluid and removing it from the nozzle. With such an arrangement, the taking up of the fluid from the nozzle permits it to be cleanly removed from the nozzle so that only a trace of the fluid is left adhered to the nozzle and will prevent any of the dispensed fluid from being drawn back into the fluid delivery member. The phrase "taking up" does not necessarily mean that the fluid is wholly accommodated in the fluid removal member and at least a portion of the fluid may be present on a surface thereof.

In order to increase the tendency for the fluid to be retained by the fluid removal member, the fluid removal member preferably includes an apertured member, which retains the fluid by surface tension. More preferably, the apertured member comprises mesh but it may alternatively comprise a foil member containing apertures made by a process such as laser piercing, a paper member wherein the paper is absorbent, or a glass mat member wherein the mat is absorbent.

For the purpose of dispensing the fluid, such as, the vegetable or plant extract mentioned above, which has a slightly oily consistency, the apertured member preferably comprises filaments having diameters in a range of 25µm to 35µm and apertures having diameters in a range of 30µm to 40µm. For fluids having different viscosities and surface tensions, however, apertured members with different characteristics may be appropriate.

Preferably, the fluid removal member includes a displacing member for moving the apertured member towards and away from the nozzle.

Since the portion of the apertured member which is used to take up the fluid will become contaminated with the fluid, the fluid removal member preferably includes a sequencing apparatus for positioning different portions of the apertured member adjacent to the nozzle in subsequent dispensing cycles.

Conveniently, the apertured member comprises an elongated member, which is displaceable, so as to position different portions thereof adjacent to the nozzle.

So as to reduce the amount of operator intervention which is required to operate the dispenser, it preferably includes a controller, which causes the delivery member to deliver the measured quantity of the fluid through the nozzle while the apertured member is closely adjacent thereto. The controller then causes the apertured member to move away from the nozzle to thereby separate the measured quantity of the fluid from the nozzle.

According to a second aspect of the invention, there is provided a method of dispensing a droplet or sub-droplet of fluid comprising forcing a measured quantity of the fluid through a nozzle, taking up the fluid with a fluid removal member positioned adjacent to the nozzle and removing the measured quantity of the fluid from the nozzle by displacing the fluid removal member away from the nozzle.

Preferably, the step of taking up the fluid involves taking the fluid into apertures in the fluid removal member and retaining it there by surface tension.

In order to remove substantially all of the fluid delivered by the nozzle, the fluid removal member preferably is placed in contact with the nozzle for the purpose of taking up the measured quantity of the fluid.

When the method is used to dispense a small dose of a vaporizable medicament, the method referred to above is preferably followed by the step of heating and vaporizing the measured quantity of the fluid. The heating is conveniently performed by passing an electrical current through at least a portion of the fluid removal member, which has taken up the measured quantity of the fluid.

According to a third aspect of the invention, there is provided a fluid measuring device including a housing with a fluid inlet and a fluid outlet, a displaceable member displaceably mounted within the housing and a floating piston slidably mounted within a bore in the displaceable member. The displaceable member is displaceable between a fill position in which a measuring chamber constituted by one end of the bore is aligned with the fluid inlet and a discharge position in which the measuring chamber is aligned with the fluid outlet. Such a device prevents contamination of a supply of the fluid connected to the fluid inlet and the floating piston can be used to repeatedly measure out precisely the same volume of the fluid while minimizing the chance of air becoming entrained in the fluid.

Preferably, the bore has a smaller diameter portion defining the measuring chamber and a relatively larger diameter portion, which respectively accommodates relatively smaller and larger piston portions. With such an arrangement, the volume of the measuring chamber can be very small while the area of the piston available for piston displacement purposes can be any convenient size.

So as to minimize the chance of the fluid or air bypassing the piston, a measuring chamber end of the piston is preferably outwardly flared for sealing engagement with an inner surface of the measuring chamber.

Conveniently, the housing includes an aperture located opposite to the fluid outlet. The dispenser further comprises a displacement member, which is insertable through the aperture to urge the piston along the bore to discharge fluid in the measuring chamber out of it.

Preferably, the displaceable member is rotatable about an axis of rotation relative to the housing so as to make the dispenser compact. The displaceable member may alternatively be longitudinally displaceable.

To simplify construction of the dispenser, an inner surface portion of the housing preferably constitutes a stop for limiting displacement of the piston as the measuring chamber is charged.

The dispenser may include a tubular reservoir for the fluid connected to the fluid inlet, a plunger sealingly engaging an inner surface of the tubular reservoir and a means for urging the piston to force the fluid from the fluid inlet into the measuring chamber. The tubular reservoir and ram may be in the form of a syringe which can be supplied in a sealed form containing a medicament.

The invention will now be described by way of example only with reference to the accompanying drawings in which:
Fig 1 shows a partly sectioned and perspective view of a dispenser according to the invention;
Fig 2 shows a view from a direction of arrow B of the dispenser shown in Fig 1;
Fig 3 shows a view from a direction of arrow C of the dispenser shown in Fig 1 with the region marked AA in Fig 2 shown in cross-section;
Fig 4 shows a plan view of a base assembly portion of the dispenser shown in Fig 1 with a moving plate assembly portion thereof removed;
Fig 5 shows a side view of a piston of the dispenser;
Fig 6 shows a side view of an electrode of the dispenser;
Fig 7 shows a side view of a core member of the dispenser;
Fig 8 shows a cross-section on the line DD of the core member shown in Fig 7;
Fig 9 shows a side view of an outer shell of the dispenser;
Figs 10 to 14 show a series of views similar to Fig 3 demonstrating how the dispenser functions; and
Fig 15 shows in schematic form a control system for controlling the dispenser.

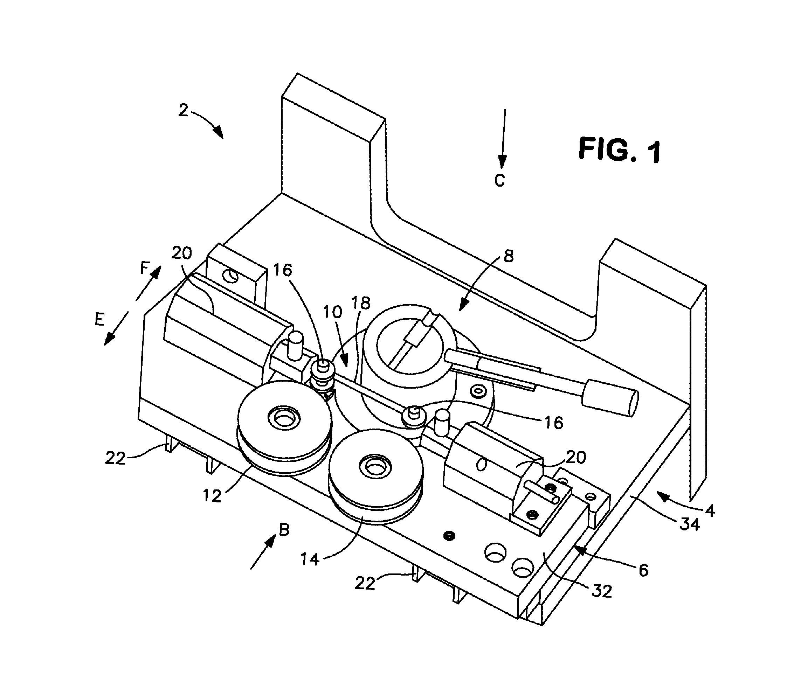

A dispenser 2 according to the invention is shown in Fig 1. The dispenser 2 includes a fixed base assembly 4 and a moving plate assembly 6, which is slidable back and forth relative to the base assembly 4 in a direction indicated by arrows E and F. The base assembly 4 includes a fluid delivery member or dosing apparatus 8, and the moving plate assembly 6 includes an apertured member 10 in the form of a strip of metal mesh which is windable past the dosing apparatus 8 from a supply reel 12 onto a take-up reel 14 around two rollers 16 between which an active portion 18 of the apertured member 10 extends. An electrode assembly 20 extends outwardly from each of the rollers 16.

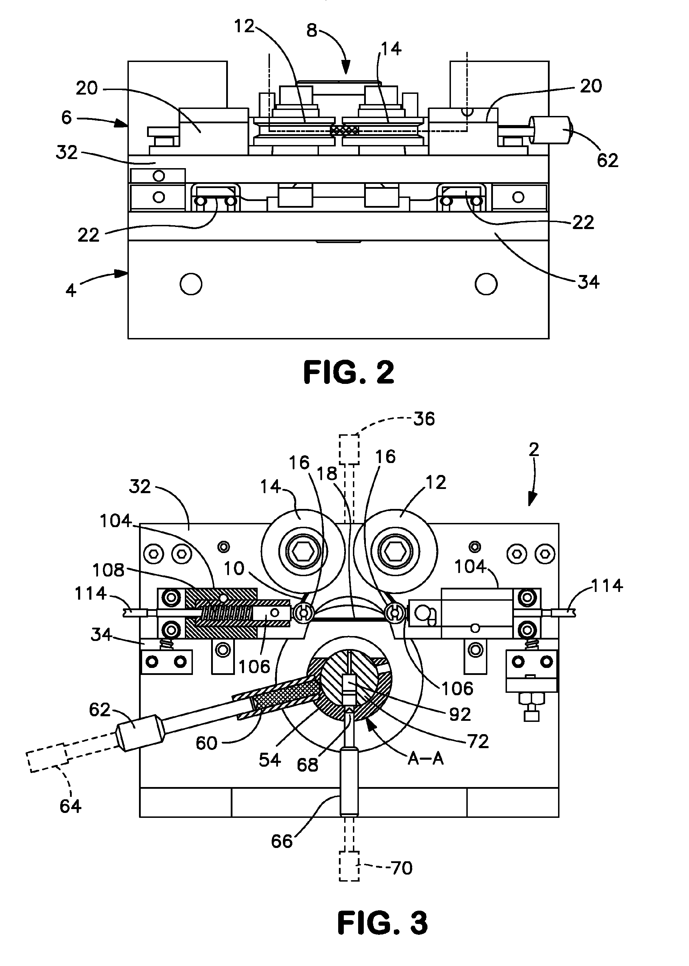

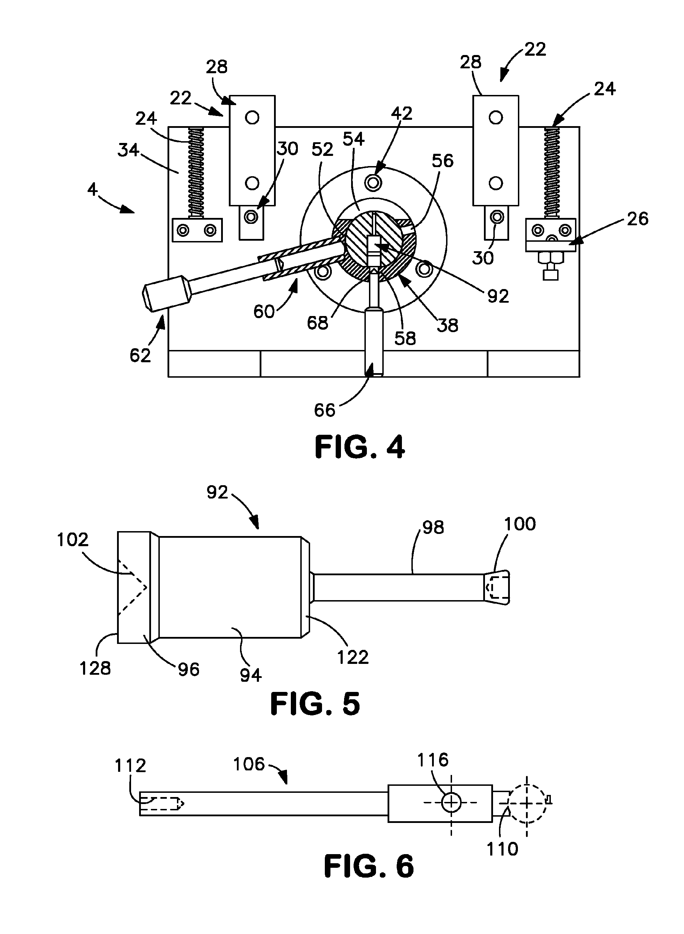

The moving plate assembly 6 is mounted on the base assembly 4 by two ball slides 22, best shown in Fig 4 where the moving plate assembly 6 has been omitted. Each of the ball slides 22 includes a first portion 28, which is connected to a moving plate 32 of the moving plate assembly 6, and a second portion 30, which is connected to a base plate 34 of the base assembly 4. Two springs 24 are also mounted on the base plate 34 by spring mounts 26. The springs 24 bias the moving plate assembly 6 in the direction of the arrow E, and a plate actuator 36 (shown with dashed lines in Fig 3 only) is provided for displacing the moving plate assembly 6 in the direction of the arrow F.

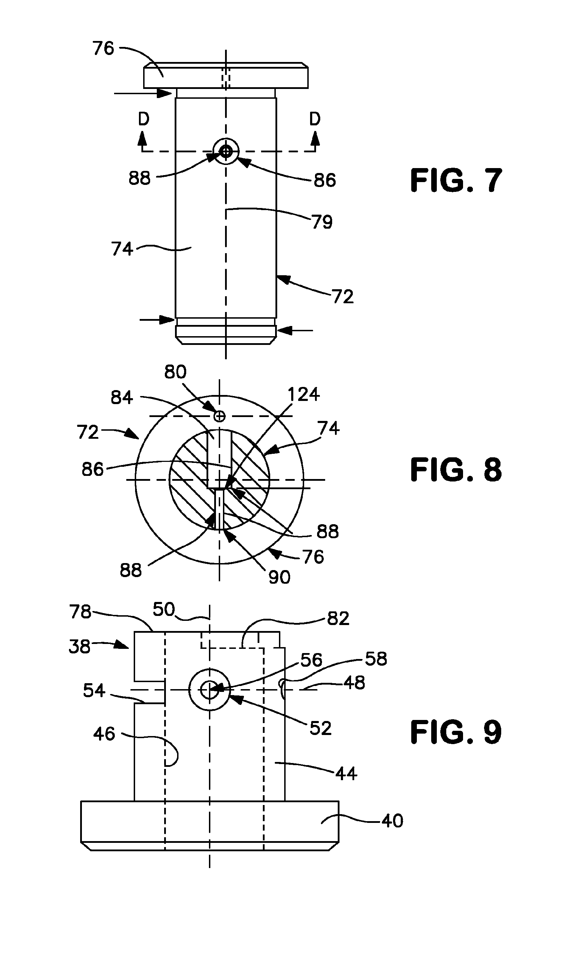

The dosing apparatus 8 will now be described in detail with reference to Figs 3 to 9 in particular. The dosing apparatus 8 includes an outer shell 38 (a side view of which is shown in Fig 9), which has a flange 40 at its lower end with which it is secured to the base plate 34 by bolts 42, an upstanding cylindrical member 44 and a bore 46. The cylindrical member 44 has four openings that are aligned on a plane 48 extending perpendicular to a central longitudinal axis 50 of the outer shell 38. The openings include an fluid inlet 52, a fluid outlet 54, a vent 56 and an actuator passage 58. As shown in Fig. 3, a tubular reservoir 60 is connected to the fluid inlet 52, and a plunger 62 slidably engages an inner surface of the tubular reservoir 60. A plunger actuator 64 is arranged to urge the plunger 62 into the tubular reservoir 60. The vent 56 is positioned diametrically opposite the fluid inlet 52, as shown in Fig. 4. An inner end 68 of a piston drive member 66 is located in the actuator passage 58, which is positioned diametrically opposite to the fluid outlet 54. A piston actuator 70 is arranged to urge the piston drive member 66 through the actuator passage 58 into the interior of the outer shell 38 and return it to the position shown in Fig 3.

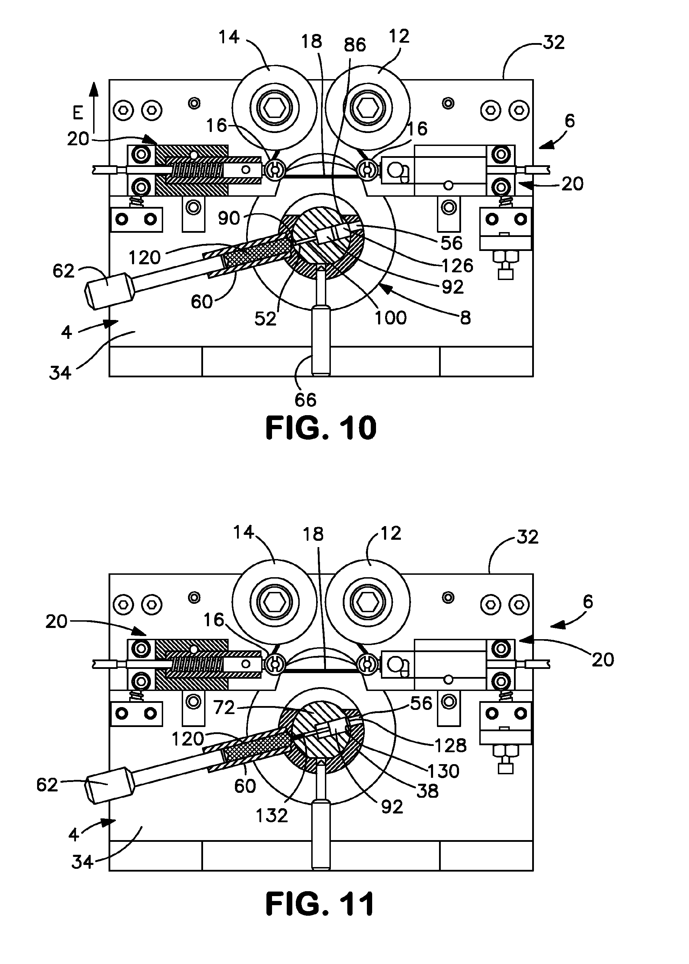

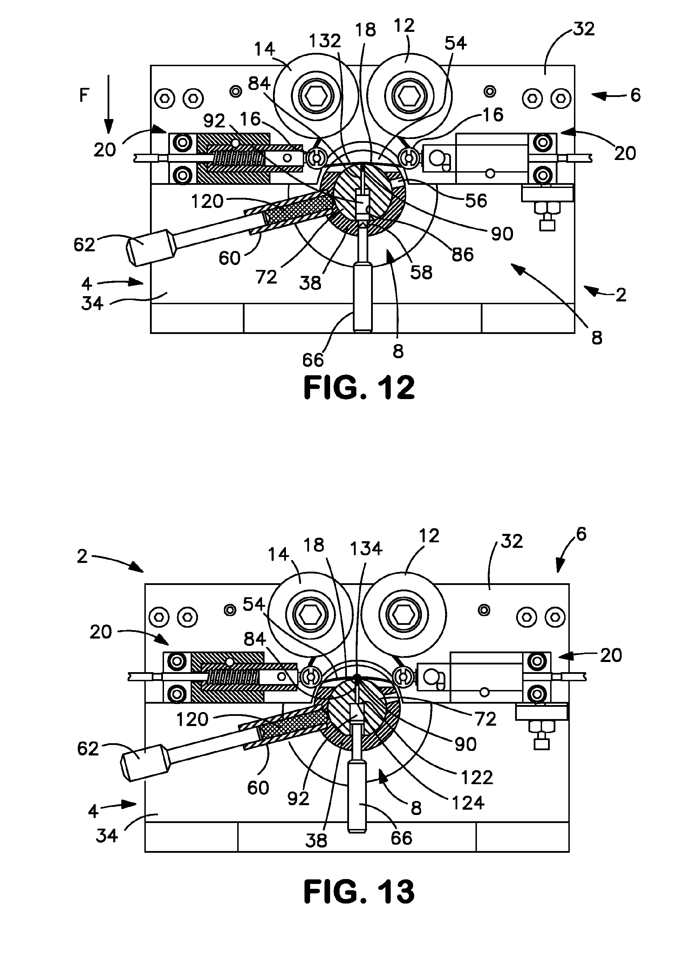

A core 72 with an outer surface 74, which is a snug sliding fit in the bore 46, is situated in the bore 46 and includes a head 76, which slidingly engages an upper end 78 of the outer shell 38, and a central longitudinal axis 79 about which it rotates. A pin 80 extends through the head 76 of the core 72 and into engagement with a pin track 82 extending part of the way around a perimeter of the upper end 78 of the outer shell 38. The movement of the pin 80 in the pin track 82 limits rotation of the core 72 in the outer shell 38 so that it can only move between the positions shown for example in Figs 11 and 12. With the core 72 so engaged with the outer shell 38, a core opening 84 in the core 72 is axially alignable with the fluid inlet 52, etc. The core opening 84 includes a relatively larger diameter portion 86, a relatively smaller diameter portion 88 and an outlet nozzle 90 at a distal end of the smaller portion 88.

A piston 92, shown in detail in Fig 5, is slidingly positioned in the core opening 84 with a larger diameter portion 94 positioned in the larger diameter portion 86 of the core opening 84 with a skirt 96 at its distal end and a smaller diameter portion 98 positioned in the smaller diameter portion 88 of the core opening 84 with an outwardly flared head 100 for sealing engagement with an inner surface of the smaller diameter portion 88 of the core opening 84. A recess 102 is provided at the distal end of the larger diameter portion 94 of the piston 92 for receiving the inner end 68 of the piston drive member 66.

The electrode assemblies 20 will now be described with reference to Figs 3 and 6. Each of the electrode assemblies 20 includes an electrode housing 104 in which an electrode 106 is slidably positioned with a tip 110 projecting towards an adjacent roller 16 and a connection end 112 projecting from an opposite end of the housing 104, which is connected to an electrical supply lead 114. An electrode spring 108 in the electrode housing 104 biases the tip 110 towards the adjacent roller 16. The radius (R) of the tip 110 matches the radius of the roller 16. The electrode 106 has a locking peg 116 projecting therefrom which is movable along an L-shaped peg track in the electrode housing 104 so as to lock the electrode 106 with the tip 110 held away from the roller 16. This may be necessary, for example, when the supply reel 12 and the take-up reel 14 are being replaced. The electrode assemblies 20 are mirror images of each other.

The supply reel 12 has a supply of the apertured member 10 wound thereonto. The active portion 18 of the apertured member 10 extends from the supply reel 12 and around the rollers 16 where the electrodes 106 nip the apertured member 10 against the rollers 16 for supplying an electrical potential across the active portion 18 of the apertured member 10. Once the apertured member 10 has been used as described below, it is wound onto the take-up reel 14. The apertured member 10 is made of steel and is composed of wires having 28µm diameters and apertures having 36µm diameters.

The operation of the dispenser 2 will now be described with particular reference to Figs 10 to 14.

The dispensing cycle is commenced with a fresh active portion 18 of the apertured member 10 extending between the rollers 16 and the moving plate assembly 6 displaced in the direction of the arrow E so that the active portion 18 is spaced from the fluid outlet 54 in the outer shell 38 and the core 72. The tubular reservoir 60 is charged with a supply of fluid 120, which is to be measured out, and the core 72 is positioned such that the nozzle 90 is lined up with the fluid inlet 52 of the outer shell 38. The piston 92 is positioned with a shoulder 122 thereof against a complementary bore shoulder 124. In this position, the head 100 of the piston 92 is situated at the nozzle 90 and a space 126 at an opposite end of the piston 92 in the larger diameter portion 86 of the core opening 84 is aligned with the vent 56.

The plunger actuator 64 then depresses the plunger 62, as shown in Fig 11, thereby forcing the fluid 120 from the supply through the fluid inlet 52 and the nozzle 90 into the smaller diameter portion 88 of the core opening 84 thereby displacing the piston 92 until an end surface 128 thereof abuts an inner surface 130 of the outer shell 38, which constitutes a piston stop. As the piston 92 moves to this position, air contained in the space 126 is exhausted through the vent 56. At this point, the portion of the smaller diameter portion 88 of the core opening 84 occupied by the fluid 120 constitutes a measuring chamber 132 and is filled with a measured volume of the fluid 120 which will be dispensed by the dispenser 2.

With reference to Fig 12, the core 72 is then rotated clockwise until the larger diameter portion 86 of the core opening 84 becomes aligned with the actuator passage 58 at which point the nozzle 90 becomes aligned with a central portion of the fluid outlet 54. At the same time, or immediately before or after such rotation, the moving plate assembly 6 is displaced in the direction of the arrow F by the plate actuator 36 so that the active portion 18 of the apertured member 10 is pressed against the nozzle 90.

The piston actuator 70 is then extended as shown in Fig. 13, which drives the piston drive member 66 through the actuator passage 58, such that its inner end 68 engages the recess 102 in the end surface 128 of the piston 92. Continued movement of the piston drive member 66 forces the piston 92 into the core opening 84 until the piston shoulder 122 contacts the core shoulder 124 thereby forcing the fluid 120 out of the measuring chamber 132, through the nozzle 90 and through the active portion 18 where it forms a bead 134 of the fluid 120.

The moving plate assembly 32 is then displaced in the direction of the arrow E by action of the plate actuator 36 and the springs 24 away from the dosing apparatus 8 thereby opening up a gap L between the active portion 18 and the core 72. Surface tension forces acting between the active portion 18 and the bead 134 droplet or sub-droplet of the dispensed fluid 120 result in substantially all of the dispensed fluid 120 remaining on or in the apertured member 10 and only a slight trace of it being left on the core 72.

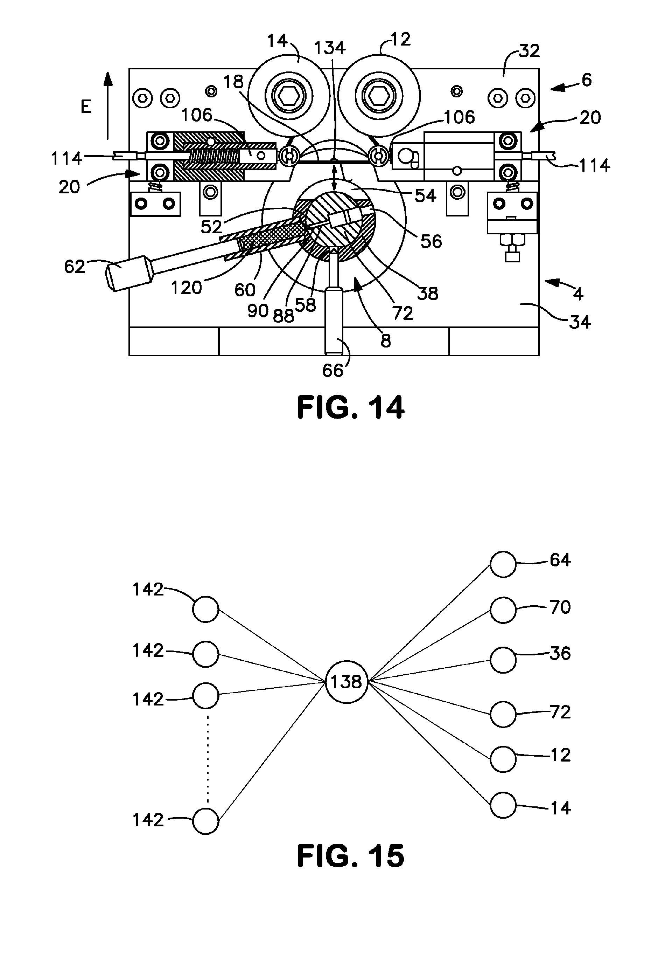

The core 72 is then rotated counter-clockwise to the position shown in Fig 14 in which the smaller diameter portion 88 of the core opening 84 is once again lined up with the fluid inlet 52 ready for the next cycle.

A voltage is then applied between the electrodes 106 via the leads 114, which results in a current flowing through the active portion 18 causing it to heat up and thereby vaporize the bead 134. Before the next cycle commences, the active portion 18 will be wound onto the take-up reel 14, which will draw a fresh active portion 18 into the region between the two rollers 16. When the dispenser 2 is being used to vaporize a medicament, it will preferably be constructed in miniaturized form and incorporated within an inhaler device.

The cycle referred to above may be carried out under the control of a controller 138, as shown in Fig 15. Inputs to the controller 138 will be provided from position sensors 142, which sense the positions of components, such as the plunger 62, the piston 92, the core 72, the supply reel 12 and the take-up reel 14. Outputs from the controller 138 will be used to control components, such as the plunger actuator 64 for displacing the plunger 62, the piston actuator 70 for displacing the piston 92, the plate actuator 36 for displacing the moving plate assembly 6, the core 72, the supply reel 12 and the take-up reel 14 and for controlling the supply of electrical current.

The moving plate assembly 6 and the fixed plate assembly 4 may be made of aluminium. The outer shell 38, the core 72, the piston 92, the plunger 62 and the tubular reservoir 60 may be made of a polyester (PET). The plate, plunger, and piston actuators 36, 64, 70 may be made of stainless steel. The supply reel 12, the take-up reel 14 and the rollers 16 may be made of a plastic material, such as TUFSET. The electrodes 16 may be made of phosphor bronze. To reduce costs in a production version, however, as many components as possible may be molded from plastic materials and additionally some combinations of the separate components described above may be formed as integral moldings.

## Claims

1. A measured droplet or sub-droplet dispenser (2) comprising a fluid delivery member (8) for measuring out a quantity of fluid including a nozzle (90) through which the quantity of fluid is delivered to the dispenser (2) further comprising a fluid removal member (6, 18) for taking up the delivered quantity of the fluid and removing it from the nozzle (90).

2. The device according to claim 1 wherein the fluid removal member includes an apertured member (18) which retains the fluid by means of surface tension.

3. The device according to claim 2 wherein the apertured member (18) comprises mesh.

4. The device according to claim 3 wherein the mesh (18) comprises filaments having diameters in the range 25µm to 35µm.

5. The device according to claim 3 or 4 wherein the mesh (18) comprises apertures having diameters in the range 30µm to 40µm.

6. The device according to any one of claims 2 to 5 wherein the fluid removal member includes a displacing member (24, 36) for moving the apertured member (18) towards and away from the nozzle (90).

7. The device according to any preceding claim wherein the fluid removal member (6, 18) includes a sequencing apparatus (12, 14) for positioning different portions of the apertured member (18) adjacent to the nozzle (90) in subsequent dispensing cycles.

8. The device according to claim 7 wherein the apertured member (18) comprises an elongated member which is displaceable so as to position different portions thereof adjacent to the nozzle (90).

9. The device according to any one of claims 2 to 8 including a controller (138) which cause the fluid delivery member (8) to deliver the measured quantity of fluid through the nozzle (90) while the apertured member (18) is closely adjacent thereto then causes the apertured member (18) to move away from the nozzle (90) to thereby separate the measured quantity of fluid from the nozzle (90).

10. A method of dispensing a droplet or sub-droplet comprising forcing a measured quantity of fluid (134) through a nozzle (90), taking up the fluid with a fluid removal member (6, 18) positioned adjacent the nozzle (90) and removing the measured quantity of the fluid (134) from the nozzle (90) by displacing the fluid removal member (6, 18) away from the nozzle (90).

11. The method according to claim 10 wherein the step of taking up the fluid involves taking the fluid into apertures in the fluid removal member (18) and retaining it there by surface tension.

12. The method according to claim 10 or 11 wherein the fluid removal member (18) is placed in contact with the nozzle (90) for the purpose of taking up the measured quantity of the fluid (134).

13. A method of dispensing a vaporizable fluid medicament involving the method according to claim 10, 11 or 12 followed by the step of heating and vaporizing the measured quantity of the fluid.

14. The method according to claim 13 wherein the heating is performed by passing an electrical current through at least a portion of the fluid removal member (18) which has taken up the measured quantity of the fluid (134).

15. A measured fluid delivery device (2) including a housing (38) with a fluid inlet (52) and a fluid outlet (54), a displaceable member (72) displaceably mounted within the housing (38) and a piston (92) slidably mounted within a bore (84) in the displaceable member (72), the displaceable member (72) being displaceable between a fill position in which a measuring chamber (132) constituted by one end of the bore (84) is aligned with the fluid inlet (52) and a discharge position in which the measuring chamber (132) is aligned with the fluid outlet (54).

16. The device of claim 15 wherein the bore (84) has a smaller diameter portion (88) defining the measuring chamber (132) and a relatively larger diameter portion (86) which respectively accommodate relatively smaller and larger piston portions (98, 94).

17. The device of claim 15 or 16 wherein a measuring chamber end (100) of the piston (92) is outwardly flared for sealing engagement with an inner surface of the measuring chamber (132).

18. The device of claim 15, 16 or 17 wherein the housing (38) includes an aperture (58) located opposite to the fluid outlet (54), the device further comprising a displacement member (66) which is insertable through the aperture (58) to urge the piston (92) along the bore (84) to discharge fluid in the measuring chamber (132) out of it.

19. The device according to any one of claims 15 to 18 wherein the displaceable member (72) is rotatable about an axis of rotation (79) relative to the housing (38).

20. The device of any one of claims 15 to 19 wherein an inner surface portion (130) of the housing (38) constitutes a stop for limiting displacement of the piston (92) as the measuring chamber (132) is charged.

21. The device according to any one of claims 15 to 20 further including a tubular reservoir (60) for the fluid (120) connected to the fluid inlet (52), a plunger (62) sealingly engaging an inner surface of the tubular reservoir (60) and means (64) for urging the plunger (62) to force fluid (120) from the fluid inlet (52) into the measuring chamber (132).
